# EUROPÄISCHE PATENTANMELDUNG

(11) **EP 0 951 864 A1**
(43) Veröffentlichungstag der Anmeldung: **27.10.1999**
(21) Anmeldenummer: 99890128.4
(22) Anmeldetag: 19.04.1999
(51) Int. Cl.: A61B 5/0408

(54) **EKG-Elektrode mit abgerundeten Ecken**

(30) Priorität: 20.04.1998 AT 25898
(71) Anmelder: Nessler Medizintechnik GmbH & Co KG, 6060 Absam (AT)
(72) Erfinder: Nessler, Norbert, Dr., 6020 Innsbruck (AT)
(74) Vertreter: Häupl, Armin, Dipl.-Ing.

(57) **Zusammenfassung**

Eine EKG-Elektrode mit einem an der Unterseite klebenden Träger (1) zum Aufkleben der Elektrode auf die Haut des Patienten, einem Kontaktknopf (2a,2b) aus leitendem Material und einer mit der Unterseite (2a) des Kontaktknopfs direkt verbundenen, vorzugsweise mit dem Träger oder einem Etikett (4) verklebten, Elektrodenzone (3) aus elektrisch leitendem Material zur Herstellung von Kontakt mit der Haut. Der Träger (1) ist in Form eines Rechtecks, insbesondere Quadrats, mit abgerundeten Ecken ausgeführt, wobei die Seitenlänge 30-35 mm und der Rundungsradius an den Ecken des Trägers (1) 5-10 mm, insbesondere 6-8 mm, beträgt.

## Beschreibung

Die Erfindung betrifft EKG-Elektroden, umfassend einen an der Unterseite klebenden Träger zum Aufkleben der Elektrode auf die Haut des Patienten, einen Kontaktknopf aus leitendem Material und eine mit der Unterseite des Kontaktknopfs direkt verbundene, vorzugsweise mit dem Träger oder einem Etikett verklebte, Elektrodenzone aus elektrisch leitendem Material zur Herstellung von Kontakt mit der Haut.

EKG-Elektroden mit dem obigen Aufbau, insbesondere Flüssiggel-Elektroden mit einem mit leitfähigem Flüssiggel getränkten Schwämmchen als Elektrodenzone, sind seit Jahren bekannt und werden weitverbreitet eingesetzt. Die einzelnen Bauteile solcher Elektroden, insbesondere das klebende Trägermaterial, sind bei gängigen Ausführungsformen allgemein kreisrund ausgeführt, konzentrisch angeordnet und miteinander verklebt und/oder vernietet. Die meisten Komponenten werden außerdem üblicherweise durch Ausschneiden oder Ausstanzen aus entsprechenden (Endlos-)Bahnmaterialien automatisiert hergestellt.

Derartige Modelle sind allerdings mit dem Nachteil behaftet, daß beim obengenannten Ausstanzen oder schneiden im "Zwickel" zwischen den einzelnen Kreisen sehr viel "Verschnitt" antällt, der verworfen wird, sich in Kosten allerdings sehr wohl niederschlägt. Zur Veranschaulichung wird darauf verwiesen, daß ein in ein Quadrat eingeschriebener Kreis nur π/4, also etwa 78,5%, der Fläche des Quadrats einnimmt, sodaß selbst bei optimaler Nutzung der Breite eines Endlosbands zum Ausstanzen von kreisförmigen Bauteilen (d.h. ohne seitlichen Rand, was in der Praxis kaum zu erreichen ist) der Material-Verschnitt etwa 21,5% beträgt. Die erhöhten Kosten schlagen sich zum Großteil beim Träger nieder, der die gesamte Querschnittsfläche der Elektrode einnimmt.

Ziel der Erfindung war daher die Reduktion der Materialkosten durch Minimierung des Verschnitts bei gleichzeitiger Gewährleistung guter Haftkraft der Elektrode an der Haut des Patienten und gutem Zusammenhalt der einzelnen Bauteile, d.h. Stabilität der Elektrode.

Erfindungsgemäß wird dieses Ziel durch eine obige EKG-Elektrode, d.h. eine EKG-Elektrode, umfassend einen an der Unterseite klebenden Träger zum Aufkleben der Elektrode auf die Haut des Patienten, einen Kontaktknopf aus leitendem Material und eine mit der Unterseite des Kontaktknopfs direkt verbundene, vorzugsweise mit dem Träger oder einem Etikett verklebte, Elektrodenzone aus elektrisch leitendem Material zur Herstellung von Kontakt mit der Haut erreicht, wobei allerdings im Gegensatz zu herkömmlichen Elektroden der Träger in Form eines Rechtecks, insbesondere Quadrats, mit abgerundeten Ecken ausgeführt ist, wobei die Seitenlänge 30-35 mm und der Rundungsradius an den Ecken des Trägers 5-10 mm, insbesondere 6-8 mm, beträgt.

Dadurch können Verschnittverluste minimiert werden, wobei aber keine Ecken verbleiben, an denen Ablösegefahr bestehen könnte. Für kreisförmige EKG-Elektroden hat sich in letzter Zeit eine Größe von wenigen cm Durchmesser bewährt. Für die erfindungsgemäßen rechteckigen/quadratischen Elektroden mit abgerundeten Ecken wurden nunmehr zahlreiche Versuchsreihen zur Optimierung der Form und der Seitenlängen- und Rundungsradius-Bereiche hinsichtlich Haftkraft/Ablösefestigkeit und Herstellungskosten durchgeführt.

Bei kürzeren Seitenlängen, d.h. unter 30 mm, ist die Haftkraft der Elektrode an der Haut des Patienten oft unzureichend. Dem könnte zwar durch eine gleichzeitige Verkleinerung der Elektrodenzone abgeholfen werden, was aber auf Kosten der elektrischen Leitung und somit der Meßgenauigkeit gehen würde. Größere Seitenlängen von über 35 mm hätten höhere Materialkosten zur Folge. Eine Vergrößerung des Rundungsradius an den Ecken auf über 10 mm würde höheren Materialverschnitt beim Ausstanzen aus Endlosbahnen mit sich bringen, während eine weitere Verkleinerung des Rundungsradius auf unter 5 mm zwar den Materialverschnitt weiter verringern und die Klebefläche erhöhen, gleichzeitig aber die Ablösefestigkeit/Haftung an den Ecken verschlechtern würde. Die quadratische Form bringt gegenüber einer rechteckigen eine zweidimensional gleichmäßigere Haftung der Elektrode an der Haut des Patienten mit sich. Aus diesen Gründen hat sich die obige Kombination aus Seitenlänge und Rundungsradius für eine quadratische Grundform als optimal herausgestellt.

Wie bei allen gängigen EKG-Elektroden kann auch bei der erfindungsgemäßen ein Etikett mit Handelsnamen, Hersteller, Spezifikationen der jeweiligen Elektrode, etc. auf dem Träger vorgesehen, vorzugsweise aufgeklebt, sein. Aus denselben Gründen, wie zuvor für den Träger beschrieben, ist das Etikett vorzugsweise ebenfalls in Form eines Rechtecks, insbesondere Quadrats, mit abgerundeten Ecken ausgeführt und insbesondere an die Form des Trägers angepaßt. Dadurch werden erfindungsgemäß auch hier Ablösefestigkeit und Materialausnutzung optimiert.

In bevorzugten Ausführungsformen umfaßt der Kontaktknopf einen Nietstift und einen Anschlußkopf jeweils aus elektrisch leitendem Material, wobei der Nietstift durch eine, vorzugsweise zentrale, Öffnung des Trägers oder des Etiketts hindurchgeführt und damit vernietet ist. Dadurch werden Etikett und Träger, gegebenenfalls zusätzlich zur Klebeverbindung, fix zusammengehalten, und gleichzeitig wird für elektrische Leitfähigkeit von der mit dem Nietstift in Kontakt stehenden Elektrodenzone zum Kontaktknopf mit den bei Gebrauch dort befestigten elektrischen Anschlüssen gesorgt.

Die Elektrodenzone kann in bekannter Weise als mit Elektrolyt, vorzugsweise Hydrogel, getränktes Schwämmchen ausgeführt sein, was gute Leitfähigkeit mit rückstandsfreier Ablösung von der Haut kombiniert. Der Nietstift besteht dabei vorzugsweise aus Silber oder versilbertem Kunststoff und ist elektrodenseitig mit einer Silberchlorid-Auflage versehen, wodurch gute und schwankungsfreie elektrische Leitung gewährleistet wird. Der Anschlußknopf kann aus Gründen der Widerstandsfähigkeit in herkömmlicher Weise aus Edelstahl gefertigt sein.

Die erfindungsgemäßen Elektroden können weiters -- wie ebenfalls auf dem Gebiet üblich -- an der Unterseite mit einer ablösbar haftenden Abziehfolie als Schutz für die Klebefläche des Trägers sowie mit einer ebenso ablösbaren Schutzabdeckung für die Elektrodenzone versehen sein, um so die Elektrode vor Gebrauch durch Beeinträchtigungen beim Hantieren oder der Lagerung zu schützen.

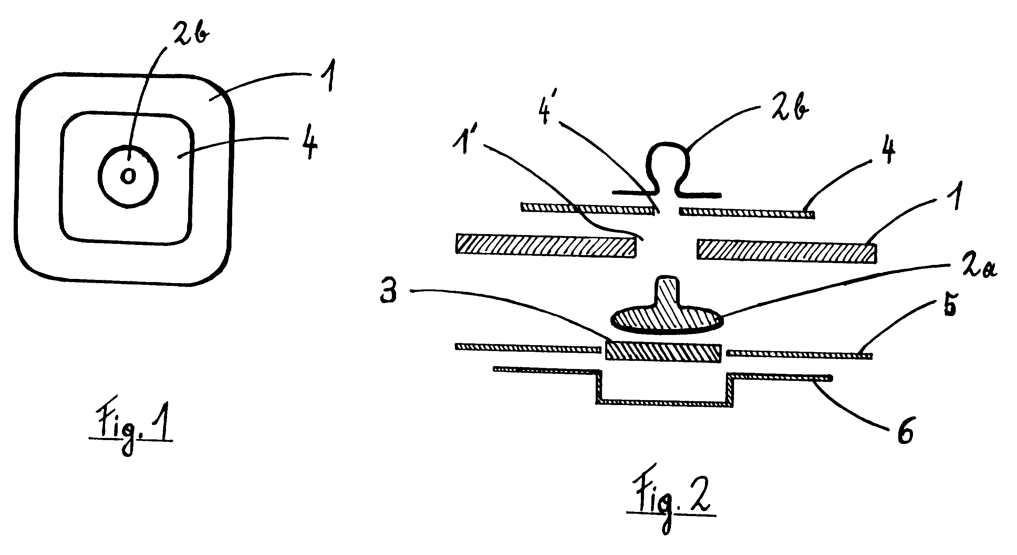

Zur Veranschaulichung des Prinzips der Erfindung sind Zeichnungen beigelegt, wovon Fig. 1 eine schematische Draufsicht auf eine bevorzugte Ausführungsform einer ertindungsgemäßen EKG-Elektrode und Fig. 2 eine schematische Explosionsansicht der EKG-Elektrode aus Fig. 1 ist.

Fig. 1 zeigt schematisch und gegenüber der Praxis etwas vergrößert eine erfindungsgemäße EKG-Elektrode als Draufsicht. Dabei ist deutlich der Träger 1 in der besonders bevorzugten Form eines Quadrats mit abgerundeten Ecken zu erkennen. Der Träger besteht herkömmlich aus einseitig (an der Unterseite) klebendem Schaumstoff und kann in einfacher Weise aus einer Endlosbahn ausgestanzt werden.

Darauf ist ein Etikett 4 aufgeklebt, dessen Form an jene des Trägers angepaßt ist. Das Etikett 4 kann den Handelsnamen des Produkts, den Namen des Herstellers, Angaben zur Elektrode oder dergleichen tragen und besteht im allgemeinen aus einseitig klebender Kunststoff-Folie.

Im Mittelpunkt von Träger 1 und Etikett 4 ist ein Kontaktknopf 2a, 2b in Form einer aus einem (in der Figur nicht dargestellten) Nietstift 2a und einem Anschlußkopf 2b bestehenden Nietverbindung angeordnet. Durch diese Nietverbindung sind Träger 1 und Etikett 4 zusätzlich zur Klebeverbindung fest miteinander vernietet.

Der Anschlußkopf 2b wird vorzugsweise aus widerstandsfähigem Edelstahl gefertigt und dient zur Befestigung der (nicht dargestellten) Anschlußkabel an der Elektrode.

Der in Fig. 2 dargestellte Nietstift 2a besteht üblicherweise aus Silber oder vorzugsweise aus Kunststoff, z.B. ABS-Polymer, mit galvanischem Silber-Überzug. Zumindest an der Unterseite ist (in Fig. 2 durch die dickere Strichführung angedeutet) eine Silberchloridschicht (durch Chlorieren des Silber-Überzugs) vorgesehen, um Schwankungen der elektrischen Leitung von Elektrodenzone 3 zum Anschlußkopf 2b auszugleichen. In der Praxis kann eine Silberchloridschicht an der gesamten Oberfläche ausgebildet sein.

Die Elektrodenzone 3 ist in Fig. 2 unterhalb des Nietstifts 2a angeordnet und in der Praxis auf das Etikett 4 aufgeklebt. Gegenüber Fig. 2 sind dann die Offnung 1' im Träger 1 und die Elektrodenzone 3 entsprechend größer, sodaß die Elektrodenzone 3 in der Offnung 1' sitzt und neben dem Nietstift 2a am Etikett 4 klebt. Vorzugsweise besteht sie aus einem mit elektrisch leitfähigem Flüssig-Gel (Hydrogel) getränkten Schwämmchen.

Der Nietstift 2a ist durch Öffnungen 1' bzw. 4' von Träger 1 und Etikett 4 hindurchgeführt, um oberhalb des Etiketts 4 in den Anschlußkopf 2b einzugreifen, wodurch die Nietverbindung hergestellt wird. In Fig. 2 ist weiters eine dünne, die Elektrodenzone 3 umgebende, ablösbare Abziehfolie 5 zum Schutz der klebenden Unterseite des Trägers 1, sowie eine zusammen mit der Folie 5 ablösbare, die Elektrodenzone 3 abschirmende Schutzabdeckung 6 dargestellt, die um die Elektrodenzone 3 herum auf die Abziehfolie 5 aufgeklebt ist.

Wie den Zeichnungen, besonders Fig. 1, zu entnehmen, wird somit erfindungsgemäß eine EKG-Elektrode bereitgestellt, die neben den üblichen Anforderungen guter Leitfähigkeit und Handhabbarkeit in material- und daher kostensparender Weise hergestellt werden kann. Zusätzlich wird durch die Vergrößerung der Haftfläche gegenüber kreisrunden Elektroden nach dem Stand der Technik mit gleichem Durchmesser die Haftung auf der Haut des Patienten verbessert, ohne daß die Gefahr des unerwünschten Ablösens von der Haut erhöht wird.

## Patentansprüche

1. EKG-Elektrode, umfassend einen an der Unterseite klebenden Träger zum Aufkleben der Elektrode auf die Haut des Patienten, einen Kontaktknopf aus leitendem Material und eine mit der Unterseite des Kontaktknopfs direkt verbundene, vorzugsweise mit dem Träger oder einem Etikett verklebte, Elektrodenzone aus elektrisch leitendem Material zur Herstellung von Kontakt mit der Haut, dadurch gekennzeichnet, daß der Träger (1) in Form eines Rechtecks, insbesondere Quadrats, mit abgerundeten Ecken ausgeführt ist, wobei die Seitenlänge 30-35 mm und der Rundungsradius an den Ecken des Trägers (1) 5-10 mm, insbesondere 6-8 mm, beträgt.

2. EKG-Elektrode nach Anspruch 1, dadurch gekennzeichnet, daß das Etikett (4) auf den Träger (1) aufgeklebt ist.

3. EKG-Elektrode nach Anspruch 1 oder 2, dadurch gekennzeichnet, daß das Etikett (4) ebenfalls in Form eines Rechtecks, insbesondere Quadrats, mit abgerundeten Ecken ausgeführt, insbesondere an die Form des Trägers (1) angepaßt, ist.

4. EKG-Elektrode nach einem der vorangegangenen Ansprüche, dadurch gekennzeichnet, daß der Kontaktknopf (2) einen Nietstift (2a) und einen Anschlußkopf (2b) jeweils aus elektrisch leitendem Material umfaßt, wobei der Nietstift (2a) durch eine, vorzugsweise zentrale, Öffnung (1', 4') des Trägers (1) oder des Etiketts (4) hindurchgeführt und damit vernietet ist.

5. EKG-Elektrode nach einem der vorangegangenen Ansprüche, dadurch gekennzeichnet, daß die Elektrodenzone (3) als mit Elektrolyt, vorzugsweise Hydrogel, getränktes Schwämmchen ausgeführt ist.

6. EKG-Elektrode nach einem der vorangegangenen Ansprüche, dadurch gekennzeichnet, daß der Nietstift (2a) aus Silber oder versilbertem Kunststoff besteht und elektrodenseitig eine Silberchlorid-Auflage aufweist.

7. EKG-Elektrode nach einem der vorangegangenen Ansprüche, dadurch gekennzeichnet, daß der Anschlußkopf (2b) aus Edelstahl besteht.

8. EKG-Elektrode nach einem der vorangegangenen Ansprüche, dadurch gekennzeichnet, daß der Träger (1) an seiner klebenden Unterseite mit einer die Elektrodenzone (3) umgebenden, ablösbaren Abziehfolie (5) versehen ist.

9. EKG-Elektrode nach einem der vorangegangenen Ansprüche, dadurch gekennzeichnet, daß die Elektrodenzone (3) mit einer ablösbaren Schutzabdeckung (6) versehen ist.
